# EUROPEAN PATENT APPLICATION

(11) **EP 1 483 975 A1**
(43) Date of publication of application: **08.12.2004**
(21) Application number: 03101639.7
(22) Date of filing: 05.06.2003
(51) Int. Cl.: A23K 1/18, A23K 1/16, A23K 1/175

(54) **Antimicrobial composition comprising a mixture of lactic acid or a derivative thereof and an inorganic acid**

(71) Applicant: PURAC Biochem BV, 4206 AC Gorinchem (NL)
(72) Inventor: Cazemier, Anne, 4143 HE, Leerdam (NL)
(74) Representative: Beetz, Tom

(57) **Abstract**

The invention relates to an antimicrobial composition comprising a mixture of lactic acid or a derivative thereof and an inorganic acid selected from a nitrogen, sulfur, and phosphorous acid, and mixtures thereof for use in animal nutrition. In one of the embodiments the composition further comprises at least one other acid selected from acetic acid, fumaric acid, gluconic acid, (iso)butyric acid, sorbic acid , (iso)valeric acid, maleic acid, malic acid, capronic acid, benzoic acid, and citric acid. In a special embodiment the composition comprises lactic acid or polylactide, which is attached to a support.

## Description

This invention relates to the use of antimicrobial compositions comprising a mixture of lactic acid or a derivative thereof and an inorganic acid, its use in a nutrition composition for animals, and its use for making a composition for treating or preventing infections in animals, especially poultry.

It is known to administer antibiotics to animals to protect them from infection by bacteria such as e.g. E. coli. It is also known to add such antibiotics to animal nutrition. However, there is an increasing resistance in using antibiotics, and many countries have legislation that prohibits the use of antibiotics in animal feed nowadays. Moreover, antibiotics are relatively expensive and they have to be administered in very controlled amounts. In order to avoid such method of protecting animals other additives for preventing infection are proposed. For instance, a composition comprising benzoic acid or a derivative of benzoic acid capable of giving rise to free benzoic acid in vivo was proposed for use as a medicament for animals in WO 96/24248. However, benzoic acid has relatively low solubility in aqueous systems. In WO 99/51218 an improvement of this method was proposed with a biocidal composition of a blend of acids substantially free of benzoic acid or a derivative thereof comprising a mixture of lactic acid and at least one other acid selected from formic acid, acetic acid and propionic acid. This blend is reported to be effective in pigs but it was now found that the blend is ineffective in poultry. Moreover, such blend with lactic acid, formic acid and propionic acid has a bad taste, and therefore cannot be used an animals that are sensitive to such taste. It is therefore an objective of the present invention to provide a composition with antimicrobial properties that is free from antibiotics and can nevertheless be effectively used in a variety of animals, including poultry, and is ingested without problems by the animal.

It was found that a composition comprising a mixture of lactic acid or a derivative thereof and an inorganic acid selected from a nitrogen, sulfur, and phosphorous acid, and mixtures thereof possesses antimicrobial properties and can be applied in animal nutrition for treating and preventing microbial infection in animals, particularly in animals with a single stomach such as pigs and rabbits, and in poultry including chicken, quail, guinea fowl, turkey, ostrich, duck, goose, pheasant, and the like.
It was further found that the present composition when used in animal nutrition is able to promote growth, improves feed to gain ratio, increase growth of lactic acid bacteria, and improve digestibility of amino acids in animal feeds, whereas the composition did not adversely affect the animal health.

With respect to the invention the term "lactic acid or a derivative thereof" means lactic acid, lactic esters, more particularly C1-C25 esters such as methyl, ethyl, ethylhexyl or octadecyl esters of lactic acid, lactylates according to Formula 1 below such as sodium stearyl lactylate and calcium lauryl lactylate, lactate and lactate salts, such as alkali metal, alkaline earth metal, ammonium, ferrous, aluminum, copper, zinc, and manganese salts of lactate, dilactate, oligolactate, and polylactate. 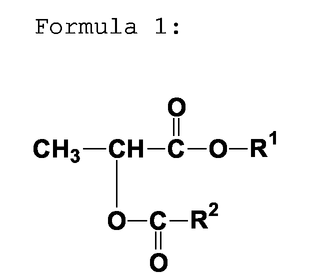 wherein R¹ stands for H or CH(CH₃)-COOH, R² stands for a C1-C35 alkyl or alkenyl group, which is either branched or unbranched;
or a Na, Ca, Fe(II), Zn, Mn, NH₄, Al, or Cu (II) salt thereof.

The inorganic acid selected from a nitrogen, sulfur, and phosphorous acid, and mixtures thereof are, for instance, nitric acid, sulfuric acid, sulfurous acid, (ortho)phosphoric acid, pyrophosphoric acid, chloric acid, and the like. The most preferred inorganic to be used in the invention is orthophosphoric acid.

The composition according to the invention preferably also comprises at least one other acid selected from acetic acid, fumaric acid, gluconic acid, (iso)butyric acid, sorbic acid , (iso)valeric acid, maleic acid, malic acid, capronic acid, benzoic acid, and citric acid. Most preferably, acetic acid is added as the additional acid. The composition may be administered to animals as a component of a conventional animal feed composition. In the context of this invention the term "animal nutrition" includes solid feed and liquid feed, such as drinking water. Thus, the antimicrobial composition may be administered to an animal as a solid or liquid component of a conventional animal feed composition or in their drinking water. It has been found very advantageous to administer the antimicrobial composition according to the invention to the drinking water of poultry, previous to their transport to the slaughterhouse as to keep them salmonella-poor.

Without being bound to the theory, it is believed that lactic acid or a derivative thereof disrupts the outer membrane of pathogens making the membrane permeable to lactic acid and other acids, such as acetic acid. The inorganic acid is believed to lower the pH in the chymus during total passage in the animal, thereby increasing the presence of non-dissociated lactic acid and other acids, such as acetic acid.

In another preferred embodiment the lactic acid or its derivative is attached to a support. This provides a convenient way to obtain the antimicrobial composition in solid powdered form. Suitable supports are selected from vegetable fiber material, vegetable carbohydrates such as cellulose, and mineral supports such as silica, starch, gypsum, and lime. Most preferably, such lactic acid (derivative) is a polylactates. Such composition is only slightly digestible and is able to enter the small intestine (duodenum) of the animal wherein it is hydrolyzed to lactic acid, thereby preventing back migration of E-coli bacteria. Preferably, the composition of this embodiment further comprises an inorganic acid, preferably orthophosphoric acid, thereby lowering the pH in the intestine, crop and gizzard, as explained hereinbefore.

The amount of lactic acid and other acids present in such a composition may vary within a wide range. Lactic acid and other suitable acids are usually sold commercially as aqueous solutions. For instance, lactic acid is sold as a 70% by weight solution whereas acetic acid is sold as a 10-80% by weight aqueous solution. Orthophosphoric acid is sold as an 85% by weight aqueous solution of phosphoric acid, and nitric acid and sulfuric acid can be obtained in concentrated as well in diluted form.

The weight ratio of lactic acid to the inorganic acid in such an antimicrobial composition is suitably in the range from 99: 1 to 1: 99, preferably from 90: 10 to 10: 90 and more preferably from 80: 20 to 20: 80.

The amount of the antimicrobial composition administered to the animal is suitably such that it is sufficient to prevent/cure any infection in the animal in its capacity as a biocide. Such an amount is suitably in the range from 0.001-5% based on the total weight of each feed fed to the animal.

This amount may, however, be higher if the function of the antimicrobial functions inter alia as an antimicrobial, promotes growth, improves feed to gain ratio, and improves digestibility of amino acids administered in animal feeds.

As mentioned above the antimicrobial composition may be administered to animals as a component of a conventional animal feed composition. A conventional animal feed composition may comprise wheat, starch, meat and bone meal, maize, sunflower meal, corn, etcetera.

The composition according to the invention may further comprise the additives composition is not only to act as an antimicrobial but also to control the pH of the animal excreta fed on such a diet in order to suppress the emission of ammonia from the excreta. Such higher amounts are suitably limited to a maximum of about 10% based on the total animal feed composition. The antimicrobial composition that are commonly added to animal nutrition, such as one or more ingredients selected from calcium salts such as carbonates, phosphates and sulfates, electrolytes, proteins, amino acids, pre-and probiotics, plant extracts, etheal oils, animal fat, vitamins and (trace) minerals.

The invention is further illustrated by the following non-limitative examples, which show the inventive merits of this invention

### General

A typical formulation for an animal feed composition is shown in the
Tables below in which all the amounts shown in % w/w were fed to chickens, wherein
Control is feed without lactic acid and orthophosphoric acid
LAFEED™ 80 is feed with 80% lactic acid without orthophosphoric acid.
LAFEED™ 80+ROPA is feed with 80% lactic acid and oregano oil.
Purac Extra is feed with 80% lactic acid, 10% orthophosphoric acid and 10% acetic acid.
Biocite™ is feed of citrus oil
Calprona™ LF is feed with 80% lactic acid and 20% formic acid.

### Animals

624 Ross 308 roosters were divided at random over 48 cages (13 roosters/cage). At an age of 10 days the number of roosters is lowered to 11 roosters per cage. The animals were vaccinated according to the following scheme.

| Scheme | | |
|---|---|---|
| age (days) | type of vaccination | administration |
| 1 | NCD/IB (Clone 30/MA5) | spray |
| 14 | Gumboro (228E, 1 dosage) | drinking water |
| 18 | NCD (Clone 30) | spray |

### Cages

The floors of the cages are provided with used litter to increase the chance to obtain infection. The room was mechanically ventilated and heated by central heating at 34° C at the start, which temperature was gradually lowered to 20° C at day 29, and thereafter maintained at 20° C. The cages were lighted by means of TL tubes. On day 1 and 2 the cages were lighted continuously, thereafter a day/night scheme of 18 hours light and 6 hours darkness was applied.

### Feed

The following feed compositions were used.

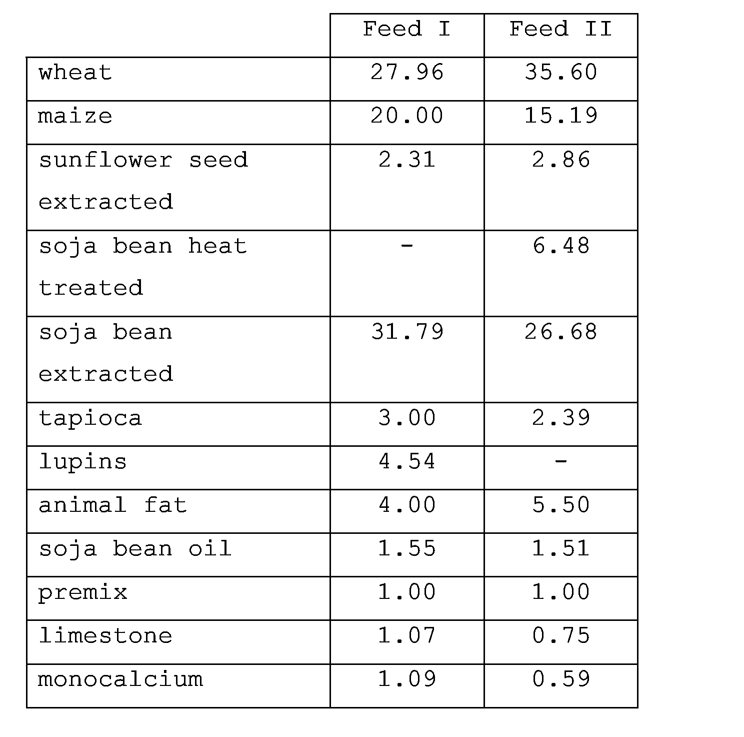

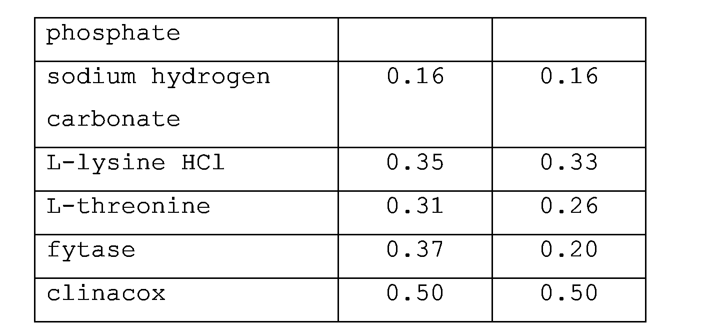

The roosters were allowed to drink and eat unlimited.

### Treatment groups

Treatment groups 1-6 were given Feed I (days 1-10) or Feed II (days 11-31) in addition to an additional composition (except Control group 1) as follows.

| treatment group | days 1-10 Feed I and additional composition | days 1-10 Feed II and additional composition |
|---|---|---|
| 1 | - | - |
| 2 | 1.5% LAFEED 80 | 0.75% LAFEED 80 |
| 3 | 1.5% LAFEED 80 + 0.05% ROPA | 0.75% LAFEED 80 + 0.025% ROPA |
| 4 | 1.5% Purac Extra | 0.75% Purac Extra |
| 5 | 0.2% Biocite | 0.2% Biocite |
| 6 | 2.0% Calprona LF | 1.0% Calprona LF |

### Performance

**Table 1.**

| Growth, mortality, feed consumption (Feed I) and feed conversion ratio in the starter period (1- 10 days) | | | | | | |
|---|---|---|---|---|---|---|
| | 1 | 2 | 3 | 4 | 5 | 6 |
| | Control | LAFEED 80 | LAFEED 80+ROPA | Purac Extra | Biocite | Calpron a LF |
| Bodyweight day 0 | 40.8 | 41.2 | 41.8 | 41.4 | 41.8 | 41.5 |
| (g) | | | | | | |
| Bodyweight day | 247 | 248 | 244 | 252 | 251 | 252 |
| 10 (g) | | | | | | |
| Growth (g) | 206 | 207 | 202 | 211 | 209 | 211 |
| Mortality (%) | 0.0 | 1.0 | 1.0 | 0.0 | 0.0 | 0.0 |
| FCR* | 1.395 | 1.401 | 1.340 | 1.339 | 1.365 | 1.376 |
| Feed consumption | 28.8 | 29.0 | 27.1 | 28.3 | 28.5 | 29.0 |
| (g/d) | | | | | | |
| Feed consumption | 288 | 290 | 271 | 283 | 285 | 290 |
| (g) | | | | | | |

| | | | | | | |
|---|---|---|---|---|---|---|
| * FCR = feed consumption ratio (= feed consumption/growth) | | | | | | |

The results of Table 1 show that with Purac Extra a low mortality combined with an optimal feed efficiency is obtained.

**Table 2.**

| Growth, mortality, feed consumption (Feed II) and feed conversion ratio in the grower period (11- 31 days) | | | | | | |
|---|---|---|---|---|---|---|
| | 1 | 2 | 3 | 4 | 5 | 6 |
| | Control | LAFEED 80 | LAFEED 80+ROPA | Purac Extra | Biocite | Calpron a LF |
| Bodyweight day | 1727 | 1740 | 1698 | 1758 | 1716 | 1715 |
| 31 (g) | | | | | | |
| Growth (g) | 1477 | 1489 | 1453 | 1504 | 1463 | 1462 |
| Mortality (%) | 1.1 | 2.3 | 1.1 | 3.4 | 3.4 | 1.1 |
| FCR | 1.652 | 1.646 | 1.661 | 1.635 | 1.698 | 1.681 |
| Feed consumption | 116.0 | 116.8 | 114.8 | 117.1 | 118.0 | 117.0 |
| (g/d) | | | | | | |
| Feed consumption | 2436 | 2452 | 2411 | 2458 | 2478 | 2457 |
| (g) | | | | | | |

The results of Table 2 show that with Purac Extra an acceptable mortality combined with an optimal feed efficiency is obtained.

## Claims

1. An antimicrobial composition comprising a mixture of lactic acid or a derivative thereof and an inorganic acid selected from a nitrogen, sulfur, and phosphorous acid, and mixtures thereof.

2. The antimicrobial composition of claim 1 wherein the inorganic acid is a phosphorous acid, preferably (ortho)phosphoric acid.

3. The antimicrobial composition of claim 1 or 2 further comprising at least one other acid selected from acetic acid, fumaric acid, gluconic acid, (iso)butyric acid, sorbic acid, (iso)valeric acid, maleic acid, malic acid, capronic acid, benzoic acid, and citric acid.

4. The antimicrobial composition of any one claim 1 - 3 wherein the lactic acid or a derivative thereof is attached to a support.

5. The antimicrobial composition of claim 4 wherein the support is selected from vegetable fiber, cellulose, silica, starch, gypsum, and lime.

6. An antimicrobial composition comprising polylactide attached to a support selected from vegetable fiber, cellulose, silica, starch, gypsum, and lime.

7. An animal nutrition composition comprising the antimicrobial composition of any one of claims 1-6.

8. Use of the composition of any one of claims 1-6 for making a nutrition composition for animals.

9. The use according to claim 8 for making a nutrition composition for poultry.

10. Use of the composition of any one of claims 1-6 for making a composition for treating or preventing microbial infection in animals.
